(19) Europäisches Patentamt
European Patent Office
Office européen des brevets

(11) EP 0 993 431 B1

(12) EUROPÄISCHE PATENTSCHRIFT

(45) Veröffentlichungstag und Bekanntmachung des Hinweises auf die Patenterteilung:
**27.03.2002 Patentblatt 2002/13**

(21) Anmeldenummer: **98937471.5**

(22) Anmeldetag: **10.06.1998**

(51) Int Cl.[7]: **C07C 41/28**, C07C 43/16

(86) Internationale Anmeldenummer:
**PCT/EP98/03521**

(87) Internationale Veröffentlichungsnummer:
**WO 98/58894 (30.12.1998 Gazette 1998/52)**

(54) **VERFAHREN ZUR HERSTELLUNG VON ENOLETHERN**
METHOD FOR PRODUCING ENOL ETHERS
PROCEDE DE FABRICATION D'ETHERS ENOLIQUES

(84) Benannte Vertragsstaaten:
**BE CH DE ES FR GB IT LI NL**

(30) Priorität: **23.06.1997 DE 19726667**

(43) Veröffentlichungstag der Anmeldung:
**19.04.2000 Patentblatt 2000/16**

(73) Patentinhaber: **BASF AKTIENGESELLSCHAFT**
**67056 Ludwigshafen (DE)**

(72) Erfinder:
- **TELES, Joaquim, Henrique**
  **D-67122 Altrip (DE)**
- **RIEBER, Norbert**
  **D-68259 Mannheim (DE)**
- **BREUER, Klaus**
  **D-67122 Altrip (DE)**
- **DEMUTH, Dirk**
  **D-68161 Mannheim (DE)**
- **HIBST, Hartmut**
  **D-69198 Schriesheim (DE)**
- **ETZRODT, Heinz**
  **D-67434 Neustadt (DE)**
- **RHEUDE, Udo**
  **D-67166 Otterstadt (DE)**

(56) Entgegenhaltungen:
**EP-A- 0 217 089** **EP-A- 0 299 286**
**EP-A- 0 776 879**

- **CHEMICAL ABSTRACTS, vol. 112, no. 7, 12. Februar 1990 Columbus, Ohio, US; abstract no. 54986a, O. N. TEMKIN: "A catalyst for the manufacture of methyl and ethyl isopropenyl ethers" Seite 687; Spalte 1; XP002080414 & DD 267 629 A**
- **CHEMICAL ABSTRACTS, vol. 112, no. 7, 12. Februar 1990 Columbus, Ohio, US; abstract no. 54987b, O. N. TEMKIN: "A catalyst for the manufacture of methyl and ethyl isopropenyl ether" Seite 687; Spalte 1; XP002080415 & DD 265 289 A**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein Verfahren zur Herstellung von Enolethern durch Komproportionierung von Ketalen bzw. Acetalen mit Alkinen oder Allenen in der Gasphase in Gegenwart eines Zink oder Cadmium und Silicium und Sauerstoff enthaltenden Heterogenkatalysators.

Es ist bekannt, Ketale oder Acetale entweder in der Flüssigphase mit sauren Katalysatoren (gemäß EP 703 211 oder EP 490 221) oder in der Gäsphase an heterogenen Katalysatoren (gemäß DE 19544450) unter Alkoholabspaltung in die entsprechenden Enolether nach der folgenden Reaktionsgleichung zu überführen:

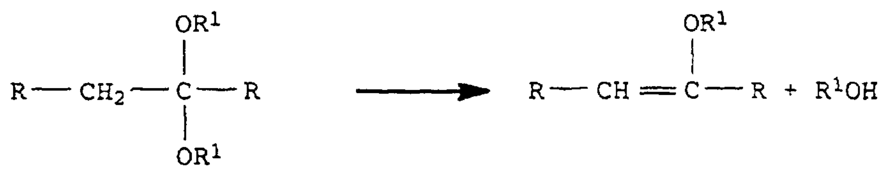

$R^1$ = Alkyl
R = Alkyl, H

Die so erhältlichen Enolether sind wichtige Ausgangsverbindungen zur Herstellung von Pharmaprodukten und Riechstoffen.

Die genannten vorbekannten Verfahren erlauben die Herstellung der Enolether zum Teil in guten Ausbeuten, haben jedoch die folgenden Nachteile:

Die Umsetzung in flüssiger Phase gemäß EP 703 211 erfordert die Verwendung einer gelösten Fremdsubstanz, nämlich einer organischen Säure, deren Entfernung aus dem Reaktionsgemisch zusätzlichen Trennaufwand erfordert und das Verfahren gemäß EP 490 221 ist nur auf Acetale anwendbar. Das Verfahren gemäß DE 19544450 hat zwar gegenüber den Verfahren in flüssiger Phase mit homogen gelöstem Katalysator den Vorteil der Reaktion in der Gasphase über einen Heterogenkatalysator, erfordert jedoch ziemlich hohe Temperaturen.

Allen diesen Verfahren ist gemeinsam, daß pro Mol Ketal-, bzw. Acetal ein Mol Alkohol freigesetzt wird, der mit zusätzlichen und z.T. erheblichem Reinigungsaufwand abgetrennt und in der Regel verworfen werden muß. Dies gilt insbesondere für Methanol, das häufig Azeotrope bildet. Die Gewichtsausbeute, bezogen auf Ketal bzw. Acetal, ist dadurch notwendigerweise vermindert.

Es bestand daher die Aufgabe, ein Verfahren vorzuschlagen, das über einen Heterogenkatalysator kontinuierlich und in guten Ausbeuten durchgeführt werden kann, sowohl auf Ketale als auch Acetale anwendbar ist und bei dem der aus dem Ketal bzw. Acetal stammende Alkohol nicht in stöchiometrischer Menge als Koppelprodukt anfällt.

Diese Aufgabe wurde erfindungsgemäß gelöst mit einem Verfahren zur Herstellung von Enolethern der Formel I

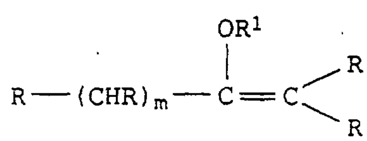

I

in der $R^1$ einen aliphatischen, cycloaliphatischen, araliphatischen, aromatischen oder heterocyclischen Rest bedeutet, wobei diese Reste weitere Substituenten, die nicht mit Acetylenen oder Allenen reagieren, tragen können und die Reste R unabhängig voneinander für Wasserstoff, oder aliphatische, cycloaliphatische, araliphatische, aromatische oder heterocyclische Reste stehen, die unter Bildung eines Ringes miteinander verbunden sein können und m für 0 oder 1 steht, bei dem man Acetale oder Ketale der Formel II

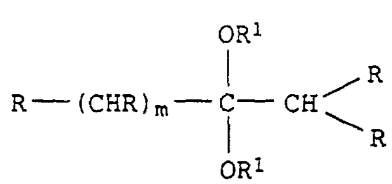

II

mit Acetylenen oder Allenen der Formeln III bzw. IV

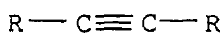 III 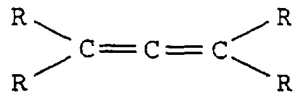 IV

wobei die Reste R und $R^1$ die oben angegebenen Bedeutungen haben, in der Gasphase in Gegenwart eines Zink oder Cadmium und Silicium und Sauerstoff enthaltenden Heterogenkatalysators umsetzt.

Obgleich der Mechanismus der erfindungsgemäßen Reaktion nicht im einzelnen bekannt ist, kann die Reaktion formal so betrachtet werden, als ob ein Mol eines Alkohols $R^1OH$ aus der Dialkoxy-Verbindung der Formel II auf das Acetylen bzw. Allen unter Bildung des Enolethers der Formel I übertragen wird.

Die als Ausgangsstoffe zu verwendenden Ketale oder Acetale der Formel II sind aus der Literatur z.B. aus US 2,667,517 und EP-A-0197283 bekannt, wobei in der Regel die beiden Reste $R^1$ identisch sind.

Die Reste R sind vorzugsweise Alkylreste, insbesondere mit 1 bis 6 C-Atomen, oder Wasserstoff und die Reste $R^1$ Alkylreste, insbesondere mit 1 bis 8 C-Atomen.

Als Acetale kommen vor allem offenkettige Verbindungen in Betracht. Beispiele für derartige Acetale sind Dimethylacetale, Diethylacetale, Di-n-propylacetale, Di-n-butylacetale, Di-i-butylacetale, Di-n-pentylacetale, Di-i-pentylacetale, Di-n-hexylacetale und Di-i-hexylacetale von Aldehyden der allgemeinen Formel

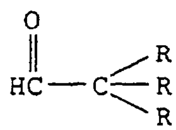

worin die Reste R die oben angegebene Bedeutung haben.

Beispiele derartiger Acetale sind:

Acetaldehyd-dimethylacetal, Acetaldehyd-diethylacetal, Acetaldehyd-dipropylacetal, Propionaldehyd-dimethylacetal, Propionaldehyd-diethylacetal, Propionaldehyd-dipropylacetal, Propionaldehyd-dibutylacetal, Butyraldehyd-dimethylacetal, Butyraldehyd-diethylacetal, Butyraldehyd-dipropylacetal, Butyraldehyd-dibutylacetal, Butyraldehyd-dipentylacetal, Valeraldehyd-dimethylacetal, Valeraldehyd-diethylacetal, Valeraldehyd-dipropylacetal, Valeraldehyd-dibutylacetal, Valeraldehyd-dipentylacetal, Isovaleraldehyd-dimethylacetal, Isovaleraldehyd-diethylacetal, Isovaleraldehyd-dipropylacetal, Isovaleraldehyd-dibutylacetal, Isovaleraldehyd-dipentylacetal, Hexanal-dimethylacetal, Hexanal-diethylacetal, Hexanal-dipropylacetal, Hexanal-dibutylacetal, Hexanaldipentylacetal, Hexanal-dihexylacetal,2-Ethylhexanal-dimethylacetal, 2-Ethylhexanal-diethylacetal, 2-Ethylhexanal-dipropylacetal, 2-Ethylhexanal-dibutylacetal, 2-Ethylhexanal-dipentylacetal, 2-Ethylhexanal-dipentylacetal, 2-Ethylhexanal-dihexylacetal und Nonanal-dimethylacetal.

Als Ketale kommen beispielsweise in Betracht:

Dimethyl-, Diethyl-, Di-n-propyl-, Di-n-butyl-, Di-i-butylketale von Aceton, Butanon-2, Pentanon-2 oder -3, von Hexanon-2 oder -3, Cyclopentanon oder von Cyclohexanon. Besonders bevorzugt als Edukt ist 2,2-Dimethoxypropan (Acetondimethylketal).

Obgleich beliebige Acetylene und Allene als Edukte gewählt werden können, verwendet man bevorzugt technisch leicht zugängliche Acetylene und/oder Allene mit 2 bzw. 3 bis 8 C-Atomen, vorzugsweise 3 bis 8 C-Atomen und insbesondere Methylacetylen oder Allen oder deren Gemische, z.B. wie sie aus einem $C_3$-Strom von einem Steamcracker isoliert werden können. Generell setzt man die Ketale bzw. Acetale bevorzugt mit solchen Acetylenen bzw. Allenen um, daß ein einheitlicher Enolether I entsteht. Dies bedeutet z.B., daß in der Formel II einer Verbindung mit m = 1 ein Allen mit den gleichen Resten R entspricht und im Falle von einer Verbindung mit m = 0 ein Acetylen mit den gleichen Resten R.

Die Umsetzung der Ketale bzw. Acetale mit den Acetylenen bzw. Allenen erfolgt in Gegenwart des heterogen vorliegenden Zink oder Cadmium und Silicium und Sauerstoff enthaltenden Katalysators in der Gasphase entweder über einem Festbett oder in einem Wirbelbett bei Temperaturen von 50 bis 400°C, vorzugsweise 100 bis 250°C und besonders bevorzugt 120 bis 200°C und Drücken von 0,1 bis 50 bar, insbesondere 0,8 bis 20 bar und besonders bevorzugt 0,9 bis 10 bar (alle Drücke bezogen auf die Summe der Partialdrücke der Edukte).

Gegebenenfalls kann das Reaktionsgemisch aus Gründen der Betriebssicherheit und der besseren Wärmeabfuhr mit Inertgasen wie Stickstoff, Argon, niedermolekularen Alkanen oder Olefinen verdünnt werden.

Das molare Verhältnis zwischen Ketal bzw. Acetal und Alkin bzw. Allen kann zwischen 0,01 und 100 liegen. Bevorzugt wird der Bereich zwischen 0,1 und 2, und besonders bevorzugt wird der Bereich zwischen 0,7 und 1,3 gewählt.

Als Zink oder Cadmium sowie Silicium und Sauerstoff enthaltende Katalysatoren kommen Cadmiumsilikate und bevorzugt Zinksilikate in Betracht, z.B. Silikate ausgewählt aus der Gruppe bestehend aus

(a) röntgenamorphen Zinksilikat oder Cadmiumsilikat, hergestellt durch Imprägnierung eines Kieselsäureträgers mit einem Zink- bzw. Cadmiumsalz,

(b) kristallinem Zinksilikat mit im wesentlichen der Zusammensetzung und Struktur des Hemimorphits der Formel $Zn_4Si_2O_7(OH)_2 \cdot H_2O$, wobei das Zink in einem bis zu 25%igen Unter- oder Überschuß, bezogen auf die stöchiometrische Zusammensetzung vorliegen kann und/oder

(c) im wesentlichen röntgenamorphen Zinksilikat, hergestellt durch Ausfällen in wäßriger Lösung aus einer löslichen Silicium- und Zinkverbindung, der Formel V

$$Zn_aSi_cO_{a-2c-0,5e}(OH)_e \cdot f\,H_2O \qquad V,$$

in der e die Werte 0 bis zur Summe aus 2a+4c bedeutet, das Verhältnis a/c 1 bis 3,5 und f/a 0 bis 200 beträgt.

(a) Röntgenamorphe Zinksilikat- oder Cadmiumsilikat-Katalysatoren werden z.B. durch Beladen von amorpher Kieselsäure mit einem Zinksalz bzw. Cadmiumsalz und Formierung des Katalysators durch eine thermische Behandlung erhalten.

Der $SiO_2$-Träger ist zumindest überwiegend amorph, hat eine BET-Oberfläche zwischen 10 und 1500 $m^2/g$, besonders bevorzugt 100 bis 500 $m^2/g$, eine Wasseraufnahmekapazität von 0,1 bis 2 ml/g, besonders bevorzugt von 0,7 bis 1,3 ml/g und kann als Pulver oder als fertiger Formkörper eingesetzt werden. Der Träger kann auch vor der Imprägnierung kalziniert werden. Bevorzugt wird der Träger aber nicht kalziniert.

Als Zink- bzw. Cadmiumverbindung verwendet man eine in einem geeigneten Lösungsmittel lösliche Verbindung. Bevorzugt werden Zink(II)-Salze verwendet, die in Wasser oder wäßrigem Ammoniak oder Alkoholen, bevorzugt niederen Alkoholen, löslich sind und deren Zersetzungstemperatur unterhalb 400°C bis 500°C liegt.

Besonders bevorzugt wird für die Imprägnierung eine ammoniakalkalische Zink(II)acetat-Lösung verwendet. In manchen Fällen hat es sich als vorteilhaft erwiesen, die Beladung mit Zink in mehreren aufeinander folgenden Tränkschritten vorzunehmen.

Wenn der Träger als Pulver eingesetzt wird, kann der Katalysator durch Formgebung (z.B. durch Mischen, Kneten und Verstrangen oder Tablettieren) in die gewünschte Form gebracht werden.

Zur Erhöhung des Porenvolumens können bei der Formgebung auch Porenbildner zugesetzt werden (z.B. Superabsorber wie Lutexal® P (Firma BASF Ludwigshafen) oder Walocel® (Methylcellulose/Kunstharz-Kombination, Firma Wolff, Walsrode)).

Alternativ kann auch ein anderer Träger, z.B. $Al_2O_3$, mit einer Siliciumoxid-Vorläuferverbindung (z.B. $Si(OR)_4$) und mit einem Zinksalz oder Cadmiumsalz imprägniert werden.

Die Zink- bzw. Cadmiumbeladung kann in weiten Grenzen variieren. Typische Werte für einen unkalzinierten Präkatalysator, der durch Tränkung eines $SiO_2$-Trägers mit einem Zinksalz bzw. Cadmiumsalz präpariert wurde, liegen z.B. zwischen 1 und 60 Gew.-% Zn oder Cd bevorzugt zwischen 7 und 30 Gew.-%. Besonders bevorzugt werden Gehalte zwischen 10 und 25 Gew.-% (jeweils berechnet als ZnO oder CdO). Der Präkatalysator kann außerdem mit anderen Elementen dotiert werden, bevorzugt mit Alkali-, Erdalkali- oder Übergangsmetallen. Ferner kann die katalytische wirksame Komponente mit bis zu 80, vorzugsweise bis zu 50 und insbesondere bis zu 20 Molprozent noch mit weiteren Metallen dotiert sein ausgewählt aus der Gruppe (A) bestehend aus Beryllium, Magnesium, Calcium, Strontium, Barium, Mangan, Eisen, Kobalt, Nickel und Kupfer, und der Gruppe (B), bestehend aus Titan, Zirkon, Hafnium, Germanium, Zinn und Blei, wobei die Elemente der Gruppe (A) teilweise Zink, bzw. Cadmium und die Elemente der Gruppe (B) teilweise Silicium ersetzen.

Der Präkatalysator kann dann bei einer Temperatur von maximal 600°C, insbesondere zwischen 80 und 300°C, an Luft oder unter Inertgas kalziniert werden. Besonders bevorzugt ist die Kalzinierung zwischen 120 und 250°C an Luft.

Nach der Herstellung des im allgemeinen katalytisch noch inaktiven Präkatalysators, durch Aufbringung einer Zink- oder Cadmium-Verbindung auf einen Siliciumoxid-Träger wird bevorzugt eine Formierung durchgeführt, bei

der sich vor allem auf der Oberfläche des Katalysators die eigentliche Aktivphase ausbildet. Diese Festkörperreaktion wird durch die Anwesenheit von Wasser, Alkoholen, bevorzugt niederen Alkoholen oder Carbonsäuren, bevorzugt niederen Carbonsäuren gefördert und wird deshalb zweckmäßigerweise durch Erhitzen des Präkatalysators bei einer Temperatur zwischen 50 und 400°C in einer wasser- oder alkoholhaltigen Atmosphäre durchgeführt. Bevorzugt führt man die Reaktion zwischen 100 und 250°C in einem wasser- oder methanolhaltigen Gasgemisch aus. Besonders bevorzugt ist die Durchführung der Reaktion zwischen 120 und 200°C mit einem methanolhaltigen Gasgemisch direkt in dem Reaktor, in dem später die Umsetzung mit dem Alkin oder Allen stattfinden soll. Wenn von einem Präkatalysator auf der Basis von Zinkacetat ausgegangen wird, kann man sehr leicht bestimmen, wann die Festkörperreaktion beendet ist, da zu diesem Zeitpunkt fast kein Methylacetat im Abgas zu finden ist. In machen Fällen hat es sich als vorteilhaft erwiesen, den Präkatalysator zur Ausbildung der Aktivphase unter Reaktionsbedingungen mit einem Gemisch aus Methanol mit Propin und Allen und eventuell auch noch anderen Komponenten (wie z.B. Propen oder Propan) zu beaufschlagen. Die Bildung der Aktivschicht wird durch das Ansteigen des Propin- und Allen-Umsatzes (nach ca. 5 bis 30 min, je nach Temperatur), durch das Ansteigen der Selektivität (nach 10 bis 300 min, je nach Temperatur) und durch das Abklingen der Konzentration von Methylacetat im Abgas angezeigt. Ein stationärer Zustand (mit hohen Propin- bzw. Allen-Umsätzen) und eine hohe Selektivität wird je nach Temperatur nach ca. 2 bis 20 Stunden erreicht.

Für die Charakterisierung der Katalysatorproben (frische, als auch Ausbauproben) wurden Standardmethoden verwendet. Die gemessene BET-Oberfläche, die typischerweise zwischen 10 und 800 $m^2/g$ liegt, sowie die Härte sind bei dem jeweiligen Beispiel angegeben. Bevorzugt werden Katalysatoren mit BET-Oberflächen zwischen 100 und 400 $m^2/g$. Weiterhin wurden die Proben mittels Pulverröntgendiffraktometrie (XRD) und Transmissionselektronenmikroskopie (TEM) eingehend untersucht. Bei beiden strukturaufklärenden Methoden ist keine Fernordnung im Sinne einer kristallinen Struktur festzustellen, sämtliche Proben waren amorph. Die Verteilung des Zinks über dem Träger wurde an entsprechenden Schnitten im Elektronenmikroskop sowie in der Mikrosonde untersucht. Sämtliche Proben zeigen, auch nach Ausbau, daß der Katalysator eine weitgehend homogene Elementverteilung besitzt und kein oder nur wenig kristallines ZnO enthält. In der IR-Untersuchung (KBr-Preßling) zeigt der mit Zinkacetat hergestellte aktive Katalysator keine Acetat-Banden (diese sind im Präkatalysator bei 1570, 1410, 670 und 610 $cm^{-1}$ noch sichtbar). Im $^{13}C$-CP-MAS-NMR sind auch keine Signale für Acetat mehr vorhanden. Im $^{29}Si$-CP-MAS-NMR zeigt der Katalysator nur die breite Bande bei -109 ppm typisch für das amorphe $SiO_2$ und eine Schulter bei -99 ppm (ca. 10 % der Intensität des Hauptpeaks). Die Elementaranalyse eines Zn-Acetat/$SiO_2$-Präkatalysators zeigt, daß das molare Verhältnis C/Zn von der Kalzinierungstemperatur abhängt. Bei Raumtemperatur getrocknete Katalysatoren haben einen C/Zn-Verhältnis von 3,5 - 4. Nach der Kalzinierung bei 200 - 250°C (optimale Temperatur) liegt das C/Zn-Verhältnis zwischen 1 und 2. Bei höheren Temperaturen sinkt das C/Zn-Verhältnis noch weiter und ebenso die katalytische Aktivität der daraus gebildeten Katalysatoren. Nach Kalzinierung bei 500°C (24 Stunden) liegt das C/Zn-Verhältnis im Präkatalysator bei 0,02. Daraus kann kein aktiver Katalysator formiert werden. Da die Zersetzung des Zinkacetats auf dem Präkatalysator relativ langsam ist, kann dieser für kurze Zeiten sogar noch höheren Temperaturen ausgesetzt werden, ohne das die katalytische Aktivität vollständig verloren geht.

(b) Hemimorphit als Katalysator

Hemimorphit ist ein Zinksilikat der Formel $Zn_4Si_2O_7(OH)_2 \cdot H_2O$. Für die erfindungsgemäße Umsetzung sind jedoch nicht nur reiner Hemimorphit, sondern allgemein heterogene Katalysatoren geeignet, die zumindest überwiegend als Aktivkomponente Zinksilikat mit der Struktur des Hemimorphits der Formel $Zn_4Si_2O_7(OH)_{2-2y}O_y \cdot x H_2O$ enthalten, wobei x und y für die Werte 0 bis 1 stehen.

Die Herstellung von Hemimorphit ist aus der Literatur bekannt. Sie kann unter Normalbedingungen oder hydrothermalen Bedingungen erfolgen.

(b1) Herstellung unter Normalbedingungen

A.G. Merkulov und B.S. Khristoforov, (Tr. Soveshch, Eksp. Tekh. Mineral. Petrogr., 8th (1971), Meeting bate 1968, 322-8; Editor(s): V.V. Lapin; Publisher: "Nauka", Moscow, USSR) beschreiben die Herstellung verschiedener Zn-Silikate durch eine Reaktion von verschiedenen Zinksalzen (Carbonat, Sulfat, Chlorid, Acetat, Oxid) mit Na-Silikat und Natronlauge in wäßriger Lösung bei Temperaturen von 90 - 100°C und bei Normaldruck. Dabei bilden sich in Abhängigkeit vom eingestellten pH-Wert unterschiedliche Zinksilikate. So entsteht reiner Sauconit mit der Zusammensetzung $Zn_3Si_4O_{10}(OH)_2 \cdot n H_2O$ bei einem End-pH-Wert von 5 - 6. Reiner Willemit ($\alpha$-$Zn_2SiO_4$) fällt im pH-Bereich von 6,5 - 8,5 an. Reiner Hemimorphit ($Zn_4Si_2O_7(OH)_2 \cdot H_2O$) kristallisiert dagegen nur im schwach alkalischen Medium bei pH-Werten von größer 10 aus.

In einer anderen Arbeit der genannten Autoren (A.G. Merkulov und B.S. Khristoforov, Izv. Sib. Otd. Akad. Nauk SSSR, Ser. Khim. Nauk (1969), (4), 70 - 4) wird angegeben, daß reiner Hemimorphit bei der Umsetzung von Zinksalzen mit Natriumsilikat und Natronlauge bei 90 - 100°C und Atmosphärendruck in wäßriger Lösung nur in

einem pH-Bereich von 10 - 12 gebildet wird.

Ferner konnten T. Baird, A.G. Cairns Smith und D.S. Snell (Reactivity of Solids, Proc. Int. Symp., 8th (1977), Göteborg, Meeting Date 1976, 337 - 42; Editor(s): J. Wood, O. Lindqvist und C. Helgesson; Publisher: Plenum Press, New York, N.Y.) große Kristalle von Hemimorphit durch Umsetzung von $Zn(OH)_2$ mit Kieselsäure und LiOH in wäßriger Lösung bei einem pH von 10 herstellen.

Schließlich wurde von H. Nagata, M. Matsunage und K. Hosokawa (Zairyo-to-Kankyo (1993) 42, 225 - 233) Hemomorphit hergestellt, indem wäßrige Zinksulfat-Lösung mit Natronlauge und wäßriger Natriumsilikat-Lösung bei einem pH von 13 umgesetzt wurde, der erhaltene Niederschlag abgetrennt, ausgewaschen und bei 85°C mindestens 24 Stunden lang gealtert wurde.

(b2) Hydrothermale Herstellung

Gemäß EP 165 647 kann Hemimorphit aus einem säurebehandelten Tonmineral und Zinkoxid bzw. Zinkhydroxid unter hydrothermalen Bedingungen (170°C, 5 h) hergestellt werden. Die Säurevorbehandlung des Tones ist jedoch sehr aufwendig und deshalb ist dieses Verfahren nachteilig.

Gemäß D.M. Roy und F.A. Mumpton (Econ. Geol. (1956) 51, 432 - 443) kann Hemimorphit ferner durch hydrothermale Umsetzung von Mischungen aus ZnO und $SiO_2$ bei 175 - 200°C gewonnen werden (Zusammensetzung: 3 ZnO + 2 $SiO_2$). Das erhaltene Produkt enthält überwiegend Hemimorphit, ist aber durch Sauconit ($Zn_3Si_4O_{10}(OH)_2 \cdot 4\ H_2O$) verunreinigt.

Schließlich beschreiben P. Taylor und D.G. Owen, (Polyhedron (1984) 3(2) 151 - 155) die hydrothermale Synthese von Hemimorphit durch Umsetzung von ZnO mit $SiO_2$ in wäßriger Lösung bei 150°C. Zur Herstellung von Produkten mit einem hohen Hemimorphit-Anteil waren jedoch lange Reaktionszeiten von mindestens 4 Tagen erforderlich.

Obgleich Hemimorphit-Produkte, die nach den oben beschriebenen bekannten Methoden erhalten wurden, sich als Katalysator für die erfindungsgemäße Addition sehr gut eignen, ergab sich, daß es wünschenswert war, deren Eigenschaften noch zu verbessern und eine Herstellungsmethode vorzuschlagen, mit deren Hilfe sich Katalysatoren mit reproduzierbar gutem Eigenschaftsprofil herstellen lassen.

Demgemäß wird eine neue Herstellungsmethode sowohl unter Normaldruck als auch unter hydrothermalen Bedingungen vorgeschlagen, bei der man ein Alkali- oder Erdalkalisilikat, vorzugsweise Natriumsilikat mit einem Zinksalz, insbesondere Zinknitrat, und einer Base wie Alkali- oder Erdalkalihydroxid, insbesondere Natriumhydroxid in wäßriger Lösung bei pH-Werten von 4 bis 9,5 vorzugsweise 5,5 bis 8 und insbesondere in einem pH-Bereich um den Neutralpunkt, z.B. bei pH 6 bis 7,5, im Falle von Normaldruck bei Temperaturen von 50 bis 100°C, insbesondere 70 bis 100°C und im Falle von hydrothermalen Bedingungen bei Temperaturen von 100 bis 250°C, bevorzugt im Bereich von 100 bis 200°C umsetzt.

Nach dieser Herstellungsmethode kann reiner Hemimorphit mit einem Zn/Si-Verhältnis von 2 synthetisiert werden. Es sind aber auch Hemimorphit-Präparate mit bis zu 25 % Unter- oder Überschuß am Zink, entsprechend einem Atomverhältnis Zn : Si von 1,6 bis 2,5 zugänglich. Als Katalysatoren werden Hemimorphite bevorzugt, die 0 bis 20 % Zinküberschuß enthalten. Besonders bevorzugt sind Hemimorphite, die 0 bis 10 % Zinküberschuß enthalten.

Die Hemimorphit-Produkte fallen bei der Synthese als weißer kristalliner Niederschlag in Form einer wäßrigen Suspension an und müssen durch geeignete Maßnahmen, z.B. Filtration oder Zentrifugieren, von der wäßrigen Lösung getrennt werden. Im Falle der Filtration wird der erhaltene Filterkuchen daraufhin Natrium- und Nitrat-frei ausgewaschen und anschließend getrocknet. Die Trocknung kann bei Temperaturen bis 600°C erfolgen, wobei der bevorzugte Temperaturbereich 90 bis 250°C umfaßt. Thermogravimetrische Untersuchungen haben gezeigt, daß der auskristallisierte Hemimorphit der Zusammensetzung $Zn_4Si_2O_7(OH)_2 \cdot H_2O$ im Temperaturbereich von etwa 100 bis 200°C unter Beibehaltung der Hemimorphit-Struktur zunehmende Anteile seines Kristallwassers verliert, wobei Hemimorphit-Präparate der Zusammensetzung $Zn_4Si_2O_7(OH)_2 \cdot x\ H_2O$ mit x kleiner als 1 resultieren, wobei x mit steigender Temperatur abnimmt. Trocknet man in einem höheren Temperaturbereich von etwa 200 bis 600°C, so werden zusätzlich, ebenfalls unter Beibehaltung der Hemimorphit-Struktur, die im Hemimorphit enthaltenen $OH^-$-Ionen in $O^{2}$-Ionen und abgespaltenes $H_2O$ umgewandelt ($2\ OH^- \rightarrow H_2O + O^{2-}$), wobei Hemimorphit-Präparate der Zusammensetzung $Zn_4Si_2O_7(OH)_{2-2y}O_y \cdot x\ H_2O$ mit y = 0 bis 1 resultieren, wobei y mit steigender Temperatur zunimmt.

Die nach der Trocknung bei Temperaturen bis 600°C, bevorzugt bei 90 bis 450°C erhaltene Hemimorphit-Präparate der Zusammensetzung $Zn_4Si_2O_7(OH)_{2-2y}O_y \cdot x\ H_2O$, wobei x und y Werte von 0 bis 1 umfaßt, werden anschließend üblicherweise mit den gebräuchlichen Formgebungsverfahren, z.B. Tablettieren, Verstrangen oder als Schalenkatalysatoren auf Steatitkugeln zu katalytischen Formkörpern verarbeitet. Einzelheiten werden in den Beispielen beschrieben.

Für die Charakterisierung der Katalysatorproben (frische Proben als auch Ausbau-Proben) wurden Standardmethoden verwendet. Die gemessene BET-Oberfläche liegt in der Regel zwischen 3 und 400 $m^2$/g. Bevorzugt

werden Katalysatoren mit BET-Oberflächen zwischen 20 und 300 $m^2/g$ verwendet. Weiterhin wurden die mit dem neuen Herstellungsverfahren erhaltenen Proben mittels Pulverröntgendiffraktometrie (XRD) und Transmissionselektronenmikroskopie (TEM) eingehend untersucht. Das gemessene Pulverröntgendiffraktogramm stimmt mit dem der Karteikarte 5-0555 der JCPDS-ICDD-Kartei (1995) überein.

(c) Röntgenamorpher Zinksilikat-Katalysator

Es wurde nun gefunden, daß man im wesentlichen unter gleichen Herstellungsbedingungen aber kürzerer Umsetzungszeit als Vorstufe zur Herstellung eines kristallinen Hemimorphits ein röntgenamorphes Produkt mit verbesserten katalytischen Eigenschaften erhält.

Dazu wird erfindungsgemäß z.B. eine wäßrige Suspension eines Alkali- oder Erdalkalisilikats mit einer wäßrigen Lösung eines Zinksalzes bei

a) Temperaturen von 20°C, bevorzugt 50°C bis zum Siedepunkt der sich ergebenden wäßrigen Suspension bei

b) einem pH-Wert von 4 bis 9,5, bevorzugt bei einem pH-Wert in der Nähe des Neutralpunktes,

c) und solchen Mengenverhältnissen von Alkalisilikat und Zinksalz umgesetzt, daß die Bedingungen der Formel VI erfüllt werden und

d) eine solche Verweilzeit eingehalten, daß noch nicht in erheblichem Maße Kristallisation des Zinksilikats eintritt.

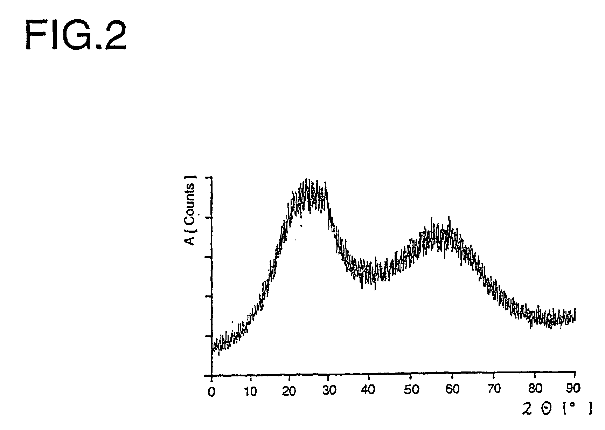

Das so erhältliche im wesentlichen röntgenamorphe Zinksilikat enthält $Zn^{2+}$-, $Si^{4+}$ und $O^{2}$-Ionen; darüber hinaus kann die Verbindung OH-Ionen und Hydratwasser enthalten. Das Zn/Si-Verhältnis beträgt 0,3 bis 5, bevorzugt 1 bis 2,7, besonders bevorzugt 2 bis 2,3 und ganz besonders bevorzugt 2. In letzterem Fall weist das röntgenamorphe Zinksilikat damit das Zn/Si-Verhältnis des kristallinen Hemimorhpits ($Zn_4Si_2O_7(OH)_2 \cdot H_2O$) auf. Das unter Anwendung von Cu-$K\alpha_1$-Strahlung ($\lambda$ = 1,5406 Å) erhaltene Pulver-Röntgenbeugungsdiagramm des erfindungsgemäßen röntgenamorphen Zinksilikats ist in Fig. 2 wiedergegeben, wobei die Intensität A der gebeugten Röntgenstrahlung als Funktion des zweifachen Beugungswinkels ($2\theta$) aufgetragen ist. Das Pulver-Röntgenbeugungsdiagramm des erfindungsgemäß zu verwendenden röntgenamorphen Zinksilikats weist im $2\theta$-Bereich von 10° bis 90° sehr breite Intensitätsmaxima bei $2\theta = 31^0 \pm 5°$ und bei $2\theta = 61° \pm 7°$ auf. In Fig. 2 sind über die genannten breiten Beugungsreflexe des erfindungsgemäß zu verwendenden röntgenamorphen Zinksilikats hinaus weitere schärfere Linien zu erkennen, die der Bildung geringer Mengen an kristallinem ZnO zugeordnet werden können (Karteikarte 5-0664 der JCPDS-ICDD-Kartei (1995)). Daneben können auch kleine Mengen an $Zn_5(NO_3)_2(OH)_8 \cdot 2\ H_2O$ auftreten (Karteikarte 24-1460 der JCPDS-ICDD-Kartei (1995)).

Auch der erfindungsgemäß zu verwendende amorphe Zinksilikat-Fällungskatalysator kann mit bis zu 80, vorzugsweise bis zu 50 und insbesondere bis zu 20 Molprozent noch mit weiteren Metallen dotiert sein ausgewählt aus der Gruppe (A) bestehend aus Beryllium, Magnesium, Calcium, Strontium, Barium, Mangan, Eisen, Kobalt, Nickel, Kupfer, Cadmium und Quecksilber und der Gruppe (B), bestehend aus Titan, Zirkonium, Hafnium, Germanium, Zinn und Blei, wobei die Elemente der Gruppe (A) teilweise Zink und die Elemente der Gruppe (B) teilweise Silicium in der Hemimorphit-Struktur ersetzen.

Das neue röntgenamorphe Zinksilikat fällt bei der Herstellung als Pulver an. Dieses Pulver kann als solches für die katalytische Umsetzung eingesetzt werden (z.B. in einem Wirbelbettreaktor) oder nach Verformung (z.B. Verstrangung, Tablettierung, etc., evtl. auch unter Zusatz von Hilfsstoffen) in einer für einen Festbettreaktor geeigneten Form.

Vor dem Einsatz kann der Katalysator bei Temperaturen zwischen 80°C und 750°C kalziniert werden. Bevorzugt wird der Katalysator zwischen 120°C und 500°C kalziniert. Besonders bevorzugt ist die Kalzinierung zwischen 200° und 400°C an der Luft. Zur Erhöhung des Porenvolumens können bei der Formgebung z.B. beim Tablettieren oder Verstrangen auch Porenbildner zugesetzt werden (z.B. Superabsorber wie Lutexal P® (Firma BASF AG) oder Walocel® (Methylcellulose/Kunstharz-Kombination, Firma Wolff, Walsrode AG)).

(A) Allgemeine Umsetzungsbedingungen

Die katalytischen Umsetzungen gemäß Fig. 1 wurden in einem gradientenfreien CSTR (Continuously Stirred Tank Reactor) (A) mit einem Volumen von 1740 ml und einem Katalysatorvolumen von ca. 90 ml, modifiziert für heterogene Gasphasenreaktionen durchgeführt. Der Reaktor hatte einen Innendurchmesser von ca. 108 mm und eine Höhe von ca. 200 mm und wurde mittels einer an der Innenwand angebrachten elektrischen Heizspirale beheizt. In

der Mitte des Reaktors war ein kleiner Zylinder aus Metall angebracht (Ø ca. 64 mm, Höhe ca. 150 mm), der auf halber Höhe (ca. 85 mm unterhalb der Oberkante) mit einem Drahtgitter versehen war. Auf dieses Drahtgitter wurde der Katalysator locker aufgeschüttet. Auf dem Deckel des Reaktors war eine flache (Ø ca. 100 mm, Höhe ca. 20 mm) Turbine angebracht, die mit 1500 - 2000 Upm angetrieben wurde. An der Reaktorachse waren auf verschiedenen Höhen insgesamt 6 Thermoelemente zur Temperaturkontrolle angebracht. Die Edukte wurden unter Druck mittels HPLC-Pumpen dosiert, kurz vor dem Reaktor gemischt und in den Reaktorraum entspannt. Das Alkin bzw. Allen (1 in Fig. 1) wurden entweder in reiner Form zudosiert oder als Gemisch mit anderen inerten Komponenten verdünnt. In dem Fall von Propin und Allen wurde ein Gemisch mit anderen Kohlenwasserstoffen eingesetzt (Zusammensetzung: 30 - 43 Vol.-% Propin, 16 - 20 Vol. -% Allen, 20 - 45 Vol.-% Propen, 5 - 10 Vol.-% Isobutan und 2 - 6 Vol.-% Propan als Hauptkomponenten; alle anderen Komponenten unter 1 %. Dieses Gemisch wird durch Destillation aus einem Seitenstrom eines Steamcrackers gewonnen. Dem Ketal (2 in Fig. 1) wurden ca. 10 Gew.-% Cyclohexan als interner Standard für die GC-Analytik zudosiert.

Die Reaktion wurde isotherm bei Temperaturen von 120 bis 300°C und einer Zulaufrate von 0,5 bis 10 mmol/min Propin und/oder Allen und 0,5 bis 20 mmol/min Ketal durchgeführt. Der Reaktionsdruck betrug 1,1 bis 3,5 bar (absolut).

Die Gesamtgasmenge, bestehend aus Edukten, Inertgas und internem Standard, betrug in der Regel zwischen 4 und 60 Nl/h. Die GHSV (gas hourly space velocity), die definiert ist als

$$\text{GHSV} = \text{Gasvolumen [Nl/h]/Katalysatorvolumen} \qquad [1]$$

betrug zwischen 80 und 1200 $h^{-1}$. Die LHSV (liquid hourly space velocity), die definiert ist als

$$\text{LHSV} = \text{Flüssigkeitsvolumen [Nl/h]/Katalysatorvolumen} \qquad [1]$$

(hier gefördertes Volumen an Propin und MeOH Volumen)
betrug zwischen 0,2 und 3 $h^{-1}$. Die Verweilzeit, definiert als Quotient aus dem katalysatorvolumen [1] und der Gasmenge [Nl/s] betrug zwischen 3 und 40 s.

Die Reaktionsgase wurden nach Verlassen des Reaktors über eine beheizte Transferleitung (3) zu einem On-line Gaschromatographen (B) geführt und dort alle 2 h analysiert. Danach wurde der Gasstrom einer partiellen Kondensation (C) unterworfen und der bei Raumtemperatur nicht kondensierbare Anteil (6) wurde in regelmäßigen Abständen (ca. 12 h) mittels Off-line GC analysiert. Das Kondensat (5) wurde ebenfalls gesammelt und mittels Off-line GC analysiert.

Wenn nichts anderes vermerkt, wurden die Umsätze und Selektivitäten auf die Summe von Propin und Allen bezogen.

Beispiel 1

a) Katalysatorherstellung (amorphes Zinksilikat; durch Imprägnierung)

Der Zn/$SiO_2$-Trägerkatalysator wurde durch Imprägnierung von röntgenamorphen $SiO_2$-Formkörpern (Kugeln mit Ø 3 - 6 mm) mit einer BET-Oberfläche von 358 $m^2$/g, Wasseraufnahmekapazität von 0,9 ml/g und Härte von 43 N/Formkörper mit ammoniakalischer Zinkacetatlösung erhalten. Dazu wurden 225 g $SiO_2$-Träger (Siligel, Fa. Solvay) mit 151,70 g $Zn(OAc)_2 \cdot 2\ H_2O$ (Merck), gelöst in 220 g 9 %iger $NH_4OH$-Lösung bei Raumtemperatur, getränkt, der Präkatalysator anschließend für 16 h bei 120°C getrocknet und dann bei 250°C für 4 h unter Luft kalziniert. Der Präkatalysator wies eine BET-Oberfläche von 195 $m^2$/g und eine Härte von 76 N/Formkörper auf. Das Acetat/Zn-Verhältnis lag bei 0,9 mol/mol.

b) Umsetzung

In der zu Fig. 1 beschriebenen Apparatur wurden ca. 90 ml des Präkatalysators eingefüllt. Propin/Allen-Gemisch (55 mol-%ig, Rest Propen) und 2,2-Dimethoxypropan wurden dann mittels HPLC-Pumpen zudosiert. Die Reaktion wurde bei der ersten Einstellung (170°C) solange fortgeführt, bis der aktive Katalysator vollständig gebildet war und Umsatz und Selektivität konstant waren (ca. 12 Stunden). Danach wurde die Temperatur und die Zuläufe gemäß Tabelle 1 geändert. Die Ergebnisse sind in Tabelle 1 zusammengefaßt. Der Druck betrug bei allen Versuchen 1,35 bar (abs). Abkürzungen: 2MP: 2-Methoxypropen; 22DMP: 2,2-Dimethoxypropan; 1MP: 1-Methoxypropen (cis und trans); 11DMP: 1,1-Dimethoxypropan. Die angegebenen Selektivitäten beziehen sich auf

Propin und Allen.

Tabelle 1

| Vers.-Nr. | Temp. °C | Zuläufe/mmol/min | | | Umsätze/% | | Selektivitäten/% | | | |
|---|---|---|---|---|---|---|---|---|---|---|
| | | Propin/ Allen | 22DMP | Gesamt | Propin/ Allen | 22DMP | 2MP | Aceton | 1MP | 11DMP |
| 1.1 | 170 | 2,31 | 2,17 | 6,20 | 69 | 90 | 95 | 1 | 2 | <1 |
| 1.2 | 150 | 1,36 | 1,57 | 4,26 | 57 | 69 | 96 | 1 | 2 | <1 |
| 1.3 | 130 | 1,13 | 1,11 | 3,31 | 44 | 59 | 96 | 1 | 2 | <1 |

Für den nach dem Ende der Versuchsreihe ausgebauten Katalysator wurde eine BET-Oberfläche von 220 $m^2/g$ und eine Härte von 74 N/Formkörper ermittelt.

Beispiel 2

a) Katalysatorherstellung (amorphes-Zinksilikat Na-dotiert)

Der $Zn/SiO_2$-Trägerkatalysator wurde durch Imprägnierung von röntgenamorphen $SiO_2$-Formkörpern (Siliperl AF125, Fa. Engelhard, Kugeln mit Ø 3 - 6 mm mit einer BET-Oberfläche von 413 $m^2/g$, Wasseraufnahmekapazität von 0,99 ml/g und Härte von 29 N/Formkörper) mit ammoniakalischer Zinkacetatlösung erhalten. Dazu wurde eine Tränklösung bestehend aus 131,46 g $Zn(OAc)_2 \cdot 2\ H_2O$ (Merck) und 2,08 g $Na(OAc) \cdot 3\ H_2O$ gelöst in einem Gemisch aus 160 g destilliertem Wasser und 120 g 25 %iger $NH_4OH$-Lösung in zwei Teile von je 195 ml geteilt und 200 g $SiO_2$-Träger bei Raumtemperatur mit dem ersten Anteil getränkt, der Präkatalysator anschließend für 16 h bei 120°C getrocknet mit dem zweiten Anteil bei Raumtemperatur getränkt, anschließend für 16 h bei 120°C getrocknet und dann bei 250°C für 4 h unter Luft kalziniert.

Der Präkatalysator wies eine BET-Oberfläche von 215 $m^2/g$ und einer Härte von 42 N/Formkörper auf und hatte die Zusammensetzung 19,5 % ZnO, 0,5 % $Na_2O$, 80 % $SiO_2$.

b) Umsetzung

In der zu Fig. 1 beschriebenen Apparatur wurden ca. 90 ml des Präkatalysators eingefüllt. Propin/Allen-Gemisch (59 mol-%ig, Rest Propen) und 2,2-Dimethoxypropan wurden dann mittels HPLC-Pumpen zudosiert. Die Reaktion wurde bei der ersten Einstellung (175°C) so lange fortgeführt, bis der aktive Katalysator vollständig gebildet war und Umsatz und Selektivität konstant waren (ca. 12 Stunden). Danach wurde die Temperatur und die Zuläufe gemäß Tabelle 2 geändert. Die Ergebnisse sind in Tabelle 2 zusammengefaßt.

Der Druck betrug bei allen Versuchen 1,3 bar (abs). Abkürzungen: 2MP: 2-Methoxypropen; 22DMP: 2,2-Dimethoxypropan; 1MP: 1-Methoxypropen (cis und Trans); 11DMP: 1,1-Dimethoxypropan. Die angegebenen Selektivitäten beziehen sich auf die Summe von Propin und Allen.

Tabelle 2

| Vers.-Nr. | Temp. °C | Zuläufe/mmol/min | | | Umsätze/% | | Selektivitäten/% | | | |
|---|---|---|---|---|---|---|---|---|---|---|
| | | Propin/ Allen | 22DMP | Gesamt | Propin/ Allen | 22DMP | 2MP | Aceton | 1MP | 11DMP |
| 2.1 | 175 | 0,90 | 1,18 | 5,52 | 85 | 88 | 95 | 2 | 2 | <1 |
| 2.2 | 160 | 2,13 | 2,51 | 12,09 | 68 | 78 | 96 | 2 | 2 | <1 |
| 2.3 | 150 | 2,13 | 2,48 | 12,01 | 58 | 63 | 96 | 2 | 1 | <1 |
| 2.4 | 135 | 1,24 | 1,21 | 6,19 | 42 | 47 | 96 | 2 | 1 | <1 |
| 2.5 | 120 | 2,00 | 2,50 | 11,84 | 35 | 29 | 96 | 3 | 1 | <1 |

Für den nach dem Ende der Versuchsreihe ausgebauten Katalysator wurde eine BET-Oberfläche von 213 $m^2/g$ und eine Härte von 45 N/Formkörper ermittelt.

Beispiel 3

a) Katalysatorherstellung (Hemimorphit Zn/Si = 2)

In einem 8-1-Rührbehälter stellte man aus 4,5 l vollentsalztem Wasser und 145,1 g pulverförmigem Natronwasserglas mit einem $SiO_2$-Gehalt von 62,1 Gew.-% und einem $Na_2O$-Gehalt von 19,0 Gew.-% (Fa. Riedel-de Haen, D-30918 Seelze) eine Suspension A mit 1,5 Mol $SiO_2$ und 0,89 Mol Na her. Ferner wurden 910,7 g $Zn(NO_3)_2 \cdot 6 H_2O$ (98 %ig) in 2,25 l vollentsalztem Wasser bei Raumtemperatur gelöst, wobei eine Lösung B mit 3 Mol Zn und 6 Mol $NO_3$ erhalten wurde. Schließlich wurde eine wäßrige Lösung aus 204,4 g NaOH in 0,225 l vollentsalztem Wasser hergestellt, wobei eine Lösung C mit einem Na-Gehalt von 5,11 Mol erhalten wurde. Nun wurden die Lösungen B und C bei Raumtemperatur in die Suspension A gegeben, wobei sich eine milchige Suspension D mit folgenden Elementanteilen ergab: Zn-Gehalt = 3 Mol, Si-Gehalt = 1,5 Mol, Na-Gehalt = 6 Mol, $NO_3$-Gehalt = 6 Mol. Der pH-Wert der erhaltenen Suspension D betrug 7,1. Die Suspension D wurde auf 90°C aufgeheizt und bei dieser Temperatur 24 Stunden lang mit einer Umdrehungszahl von 200 Upm gerührt. Die Suspension wurde anschließend auf Raumtemperatur abgekühlt und ein End-pH-Wert von 7,0 gemessen. Der auskristallisierte weiße Niederschlag wurde abfiltriert und mit vollentsalztem Wasser Na-frei gewaschen und der anfallende Filterkuchen bei 90°C in einem Trockenschrank getrocknet.

Das getrocknete weiße Pulver wurde röntgenographisch untersucht, wobei sich ein Röntgenpulverdiagramm ergab, das dem der Karteikarte 5-0555 der JCPDS-ICDD-Kartei (1995) voll entsprach und damit der Herstellung von $Zn_4Si_2O_7(OH)_2 \cdot H_2O$ anzeigte. Die nach BET bestimmte spezifische Oberfläche des erhaltenen Pulvers betrug 30 $m^2/g$.

Zur Herstellung eines Katalysators wurde das noch feuchte Pulver direkt zu Strängen verpreßt (Ø = 3 mm, Preßdruck = 50 bar), die anschließend 16 h bei 120°C getrocknet wurden. Der fertige Katalysator hatte eine BET-Oberfläche von 26 $m^2/g$ und eine Härte von 6 N/Formkörper.

b) Umsetzung

In der oben beschriebenen Apparatur wurden ca. 90 ml des Katalysators eingefüllt. Propin/Allen-Gemisch (ca. 63 vol-%ig, 1,68 mmol/min) und 2,2-Dimethoxypropan (2,17 mmol/min); Gesamtzulauf mit Inerten: 6,46 mmol/min; Verhältnis 2,2-Dimethoxypropan/(Propin+Allen) = 1,29) wurden dann mittels HPLC-Pumpen zudosiert. Die Reaktionstemperatur betrug 170°C und der Druck 1,35 bar (abs), der Partialdruck der Edukte betrug 0,8 bar. Es wurden von Anfang an (d.h. der Katalysator hat keine Formierungszeit) folgende Selektivitäten beobachtet: 2-Methoxypropen: 97,4 %; Aceton: 2,3 %; cis und trans 1-Methoxypropen: 0,3 %.

Für den Katalysator wurden nach dem Ausbau die folgenden Werte ermittelt: BET 25 $m^2/g$, Härte von 6 N/Formkörper.

Beispiel 4

a) Katalysatorherstellung (Hemimorphit Zn/Si = 2,2)

In einem 6 l Rührbehälter wurde aus 3,0 l vollentsalztem Wasser und 96,8 g pulverförmigem Natronwasserglas mit einem $SiO_2$-Gehalt von 62,1 Gew.-% und einem $Na_2O$-Gehalt von 19,0 Gew.-% (Fa. Riedel-de Haen, D-30918 Seelze) eine Suspension A mit 1,0 Mol $SiO_2$ und 0,59 Mol Na und aus 667,8 g $Zn(NO_3)_2 \cdot 6 H_2O$ (98 %ig) in 1,5 l vollentsalztem wasser bei Raumtemperatur eine Lösung B mit 2,2 Mol Zn und 4,4 Mol $NO_3$ sowie eine wäßrige Lösung C mit einem Na-Gehalt von 3,81 Mol aus 152,3 g NaOH in 0,4 l vollentsalztem Wasser hergestellt. Die Lösungen B und C wurden in die Suspension A bei Raumtemperatur gegeben, wobei sich eine milchige Suspension D mit folgenden Elementanteilen ergab: Zn-Gehalt = 2,2 Mol, Si-Gehalt = 1 Mol, Na-Gehalt = 4,4 Mol, $NO_3$-Gehalt = 4,4 Mol. Der pH-Wert der erhaltenen Suspension D betrug 7,2. Die Suspension D wurde auf 90°C erhitzt und bei dieser Temperatur 24 Stunden lang mit einer Umdrehungszahl von 200 Upm gerührt. Nach Abkühlen der Suspension auf Raumtemperatur wurde ein End-pH-Wert von 7,0 gemessen. Der angefallene weiße Niederschlag wurde abfiltriert, mit vollentsalztem Wasser Na-frei gewaschen und der erhaltene Filterkuchen bei 90°C in einem Trockenschrank getrocknet.

Das getrocknete weiße Pulver wurde röntgenographisch untersucht, wobei sich ein Röntgenpulverdiagramm ergab, das dem der Karteikarte 5-0555 der JCPDS-ICDD-Kartei (1995) voll entsprach und damit die Herstellung

von $Zn_4Si_2O_7(OH)_2 \cdot H_2O$ anzeigte. Die nach BET bestimmte spezifische Oberfläche des erhaltenen Pulvers betrug 60 $m^2$/g.

650 g des Pulvers wurden mit 20,2 g Magnesiumstearat (Merck) vermischt und zu 20 mm Tabletten gepreßt. Diese Tabletten wurden zu Splitt (< 0,5 mm) verarbeitet. Die Tabletten wurden dann 10 Stunden bei 350°C kalziniert. Der fertige Katalysator hatte eine BET-Oberfläche von 44 $m^2$/g und eine Härte von 44 N/Formkörper.

b) Umsetzung

In der oben beschriebenen Apparatur wurden ca. 90 ml des Katalysators eingefüllt. Propin/Allen-Gemisch (ca. 60 vol-%ig, 1,64 mmol/min) und 2,2-Dimethoxypropan (2,14 mmol/min; Gesamtzulauf mit Inerten: 8,34 mmol/min; Verhältnis 2,2-Dimethoxypropan/(Propin+Allen) = 1,30) wurden dann mittels HPLC-Pumpen zudosiert. Die Reaktionstemperatur betrug 170°C und der Druck 1,35 bar (abs.), der Partialdruck der Edukte betrug 0,8 bar. Es wurden von Anfang an (d.h. der Katalysator hat keine Formierungszeit) folgenden Selektivitäten beobachtet: 2-Methoxypropen: 97,0 %; Aceton: 2,3 %; cis und trans 1-Methoxypropen: 0,5 %.

Für den Katalysator wurden nach dem Ausbau die folgenden Werte ermittelt: BET 44 $m^2$/g, Härte von 12 N/ Formkörper.

Beispiel 5

(Röntgenamorpher Fällungskatalysator; Zn/Si-Verhältnis 2,1)

In einem 12-1-Rührbehälter wurden unter ständigem Rühren (100 Upm) in 7,5 l vollentsalztem und 80°C warmem Wasser 120,93 g pulverförmiges Natronwasserglas mit einem $SiO_2$-Gehalt von 62,1 Gew.-% und einem $Na_2O$-Gehalt von 19,0 Gew.-% (Fa. Riedel-de-Haen, D-30918 Seelze) gegeben, wobei eine Suspension A mit 1,25 Mol $SiO_2$ und 0,74 Mol Na erhalten wurde. Anschließend wurde eine wäßrige Lösung B aus 180,4 g NaOH (entspricht 4,51 Mol Na) in 0,5 l vollentsalztem Wasser hergestellt. Außerdem wurden 796,8 g $Zn(NO_3)_2 \cdot 6\ H_2O$ (Zn-Gehalt = 98 %) in 2,5 l vollentsalztem Wasser gelöst, wobei eine Lösung C mit 2,625 Mol Zn und 5,25 Mol $NO_3$ erhalten wurde. Daraufhin wurde die Lösung B in die 80°C warme Suspension A gegeben, wobei nach ca. 5 Minuten eine klare Lösung D erhalten wurde. In die erhaltene Lösung D wurde anschließend die Lösung C gegeben. Dabei ergab sich eine weiße Suspension E mit einem Zn-Gehalt von 2,625 Mol, einem Si-Gehalt von 1,25 Mol, einem Na-Gehalt von 5,25 Mol und einem $NO_3$-Gehalt von 5,25 Mol. Die Suspension E wurde bei 80°C 2 Stunden lang unter Rühren (100 Upm) erhitzt und anschließend auf Raumtemperatur abgekühlt. Es wurde nach Abkühlung ein End-pH-Wert von 6,5 gemessen. Der angefallene weiße Niederschlag wurde abfiltriert und mit vollentsalztem Wasser Na-frei gewaschen. Der erhaltene Filterkuchen wurde bei 80°C in einem Trockenschrank getrocknet.

Das getrocknete weiße Pulver wurde röntgenographisch untersucht, wobei sich ein Röntgenpulverdiagramm ergab, das dem der Fig. 2 entspricht und damit die Herstellung von überwiegenden Mengen an röntgenamorphem Zinksilikat neben einer geringen Menge an kristallinem ZnO (Karteikarte 5.0664 der JCPDS-ICDD-Kartei (1995)) anzeigte. Die nach 3ET bestimmte spezifische Oberfläche des erhaltenen Pulvers betrug 102,1 $m^2$/g.

650 g des wie oben beschrieben hergestellten amorphen Zinksilikats mit der Zusammensetzung von Hemimorphit wurde wurden mit 20,2 g Zn-Stearat vermischt und zu 20-mm-Tabletten vorkompaktierc, anschließend zu Splitt mit einem Durchmesser von < 0,5 mm zerkleinert und dann zu Tabletten mit dem Format 4,75 x 5,2 mm verformt. Der Katalysator besaß eine BET-Oberfläche von 75 $m^2$/g und eine Härte von 43 N/Tablette. Eine 100 g Portion des Katalysators wurde dann bei 350°C für 10 Stunden an der Luft kalziniert.

In der oben beschriebenen Apparatur wurden ca, 90 ml des Katalysators eingefüllt. Propin/Allen-Gemisch (49,8 vol.-%ig, 1,84 mmol/min) und 2,2-Dimethoxypropan (2,15 mmol/min; Gesamtzulauf mit Inerten: 6,79 mmol/min; Verhältnis 2,2-Dimethoxypropan/(Propin+Allen) = 1,17) wurden dann mittels HPLC-Pumpen zudosiert. Die Reaktionstemperatur betrug 170°C und der Druck 1,35 bar (abs), der Partialdruck der Edukte 0,8 bar. Es wurden folgende Selektivitäten beobachtet: 2-Methoxypropen 97,1 %; 2-Dimethoxypropan 2,5 %, cis- und trans-1-Methoxypropen 0,4 %. Der Umsatz, bezogen auf Propin/Propadien, betrug 21 %.

Dieser Katalysator zeigt praktisch keine Formierungszeit. Der angegebene Umsatz und die Selektivität waren von Anfang an quasi gleichbleibend, Die BET-Oberfläche betrug nach der Kalzinierung 82 $m^2$/g und nach dem Ausbau 64 $m^2$/g. Die Härte betrug nach der Kalzinierung 28 N/Tablette und nach dem Ausbau 36 N/Tablette.

**Patentansprüche**

1. Verfahren zur Herstellung von Enolethern der Formel I

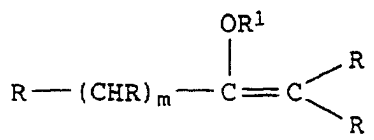 I

in der $R^1$ einen aliphatischen, cycloaliphatischen, araliphatischen, aromatischen oder heterocyclischen Rest bedeutet, wobei diese Reste weitere Substituenten, die nicht mit Acetylenen oder Allenen reagieren, tragen können und die Reste R unabhängig voneinander für Wasserstoff, oder aliphatische, cycloaliphatische, araliphatische, aromatische oder heterocyclische Reste stehen, die unter Bildung eines Ringes miteinander verbunden sein können und m für 0 oder 1 steht, **dadurch gekennzeichnet, daß** man Acetale oder Ketale der Formel II

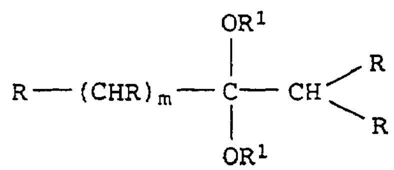 II

mit Acetylenen oder Allenen der Formeln III bzw. IV

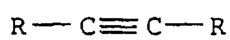 III 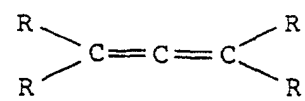 IV

wobei die Reste R und $R^1$ die oben angegebenen Bedeutungen haben, in der Gasphase bei erhöhter Temperatur in Gegenwart eines Zink oder Cadmium zusammen mit Silicium und Sauerstoff enthaltenden Heterogenkatalysators umsetzt.

2. Verfahren gemäß Anspruch 1, **dadurch gekennzeichnet, daß** man die Umsetzung in Gegenwart von Katalysatoren durchführt, die eine BET-Oberfläche von 10 bis 800 $m^2/g$ aufweisen.

3. Verfahren gemäß Anspruch 1, **dadurch gekennzeichnet, daß** man als Katalysator ein röntgenamorphes Zinksilikat verwendet, erhältlich durch Aufbringen eines Zinksalzes auf amorphe Kieselsäure und Formierung des Katalysators bei 50 bis 400°C.

4. Verfahren gemäß Anspruch 1, **dadurch gekennzeichnet, daß** man ein Zinksilikat mit Hemimorphit-Struktur der Formel $Zn_4Si_2O_7(OH)_{2-2y}O_y \cdot xH_2O$ verwendet, wobei x und y für die Werte 0 bis 1 stehen.

5. Verfahren gemäß Anspruch 1, **dadurch gekennzeichnet, daß** man als Katalysator ein durch Ausfällung in wässriger Lösung erhältliches im wesentlichen röntgenamorphes Zinksilikat der Formel V

$$Zn_aSi_cO_{a+2c-0,5e}(OH)_e \cdot f\,H_2O \qquad V$$

verwendet, in der e die Werte 0 bis zur Summe aus 2a+4c bedeutet und das Verhältnis a/c 1 bis 3,5 und f/a 0 bis 200 beträgt.

6. Verfahren gemäß Anspruch 1, **dadurch gekennzeichnet, daß** man Ketale bzw. Acetale der Formel II mit solchen Acetylenen bzw. Allenen der Formeln III und IV umsetzt, daß 2 Mol des gleichen Enolethers I erhalten werden.

7. Verfahren gemäß Anspruch 1, **dadurch gekennzeichnet, daß** man 2-Methoxypropen durch Umsetzung von 2,2-Dimethoxypropan mit Methylacetylen und/oder Allen herstellt.

**8.** Verfahren gemäß Anspruch 1, **dadurch gekennzeichnet, daß** man die Umsetzung bei Temperaturen von 50 bis 400°C und einem Druck von 0,1 bis 50 bar durchführt.

## Claims

**1.** A process for the preparation of enol ethers of the formula I

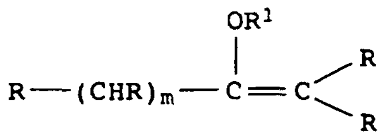 I

where $R^1$ is an aliphatic, cycloaliphatic, araliphatic, aromatic or heterocyclic radical which may carry further substituents which do not react with acetylenes or allenes, and the radicals R, independently of one another, are hydrogen or aliphatic, cycloaliphatic, araliphatic, aromatic or heterocyclic radicals, which may be bonded to one another to form a ring, and m is 0 or 1, wherein an acetal or ketal of the formula II

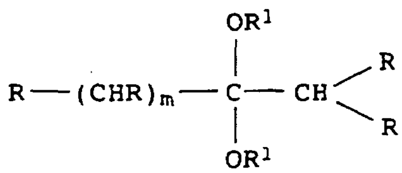 II

is reacted with an acetylene or allene of the formula III or IV

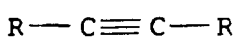 III 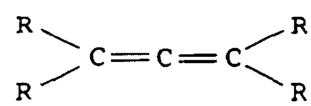 IV

where R and $R^1$ have the abovementioned meanings, in the gas phase at elevated temperatures in the presence of a zinc- or cadmium- and silicon- oxygen-containing heterogeneous catalyst.

**2.** A process as claimed in claim 1, wherein the reaction is carried out in the presence of a catalyst which has a BET surface area of from 10 to 800 $m^2/g$.

**3.** A process as claimed in claim 1, wherein the catalyst used is an X-ray amorphous zinc silicate obtainable by applying a zinc salt to amorphous silica and forming the catalyst at from 50 to 400°C.

**4.** A process as claimed in claim 1, wherein a zinc silicate having the hemimorphite structure of formula $Zn_4Si_2O_7(OH)_{2-2y}O_y \cdot xH_2O$, where x and y are each from 0 to 1, is used.

**5.** A process as claimed in claim 1, wherein the catalyst used is an essentially X-ray amorphous zinc silicate obtainable by precipitation in aqueous solution and of the formula V

$$Zn_aSi_cO_{a+2c-0.5e}(OH)_e \cdot f\,H_2O \qquad V,$$

where e is from 0 to 2a+4c and the ratio a/c is from 1 to 3.5 and the ratio f/a is from 0 to 200.

**6.** The process as claimed in claim 1, wherein a ketal or acetal of the formula II is reacted with an acetylene or allene of the formula III or IV such that 2 mol of the same enol ether I are obtained.

**7.** A process as claimed in claim 1, wherein 2-methoxypropene is prepared by reacting 2,2-dimethoxypropane with methylacetylene and/or allene.

**8.** A process as claimed in claim 1, wherein the reaction is carried out at from 50 to 400°C and from 0.1 to 50 bar.

**Revendications**

**1.** Procédé pour la préparation d'éthers d'énols de formule I

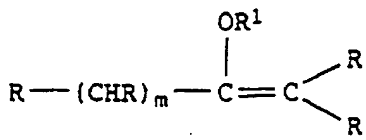

I

dans laquelle $R^1$ représente un radical aliphatique, cycloaliphatique, araliphatique, aromatique ou hétérocyclique, chacun de ces radicaux pouvant porter d'autres substituants qui ne réagissent pas avec les acétylènes ou les allènes, et les symboles R représentent chacun, indépendamment les uns des autres, l'hydrogène ou un radical aliphatique, cycloaliphatique, araliphatique, aromatique ou hétérocyclique qui peuvent être reliés entre eux avec formation d'un cycle, et m est égal à 0 ou 1, **caractérisé par le fait que** l'on fait réagir des acétals de formule II

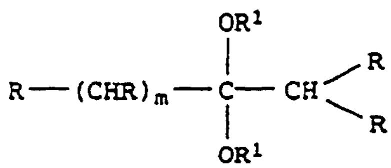

II

avec des acétylènes ou des allènes de formules respectives III et IV

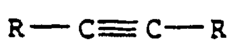

III

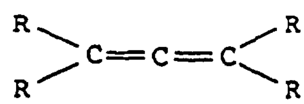

IV

dans lesquelles les symboles R et $R^1$ ont les significations indiquées ci-dessus, en phase gazeuse, à température élevée, en présence d'un catalyseur hétérogène contenant du zinc ou du cadmium avec du silicium et de l'oxygène.

**2.** Procédé selon la revendication 1, **caractérisé par le fait que** l'on procède à la réaction en présence de catalyseurs ayant une surface BET de 10 à 800 $m^2/g$.

**3.** Procédé selon la revendication 1, **caractérisé par le fait que** l'on utilise en tant que catalyseur un silicate de zinc amorphe aux rayons X, obtenu par application d'un sel de zinc sur une silice amorphe suivie d'un finissage du catalyseur à des températures de 50 à 400°C.

**4.** Procédé selon la revendication 1, **caractérisé par le fait que** l'on utilise un silicate de zinc à structure d'hémimorphite de formule $Zn_4Si_2O_7(OH)_{2-2y}O_y.xH_2O$ dans laquelle x et y ont des valeurs de 0 à 1.

**5.** Procédé selon la revendication 1, **caractérisé par le fait que** l'on utilise en tant que catalyseur un silicate de zinc essentiellement amorphe aux rayons X obtenu par précipitation en solution aqueuse et répondant à la formule V

$$Zn_aSi_cO_{a+2c-0,5e}(OH)_e \cdot f\,H_2O \qquad V$$

dans laquelle e est compris entre 0 et la somme 2a+4c, le rapport a/c va de 1 à 3,5 et le rapport f/a va de 0 à 200.

**6.** Procédé selon la revendication 1, **caractérisé par le fait que** l'on fait réagir des acétals de formule II avec des acétylènes ou des allènes de formules respectives III et IV de manière à obtenir 2 mol du même éther d'énol I.

**7.** Procédé selon la revendication 1, **caractérisé par le fait que** l'on prépare le 2-méthoxy-propène par réaction du 2,2-diméthoxypropane avec le méthylacétylène et/ou l'aliène.

**8.** Procédé selon la revendication 1, **caractérisé par le fait que** l'on procède à la réaction à des températures de 50 à 400°C sous une pression de 0,1 à 50 bar.

Fig. 1

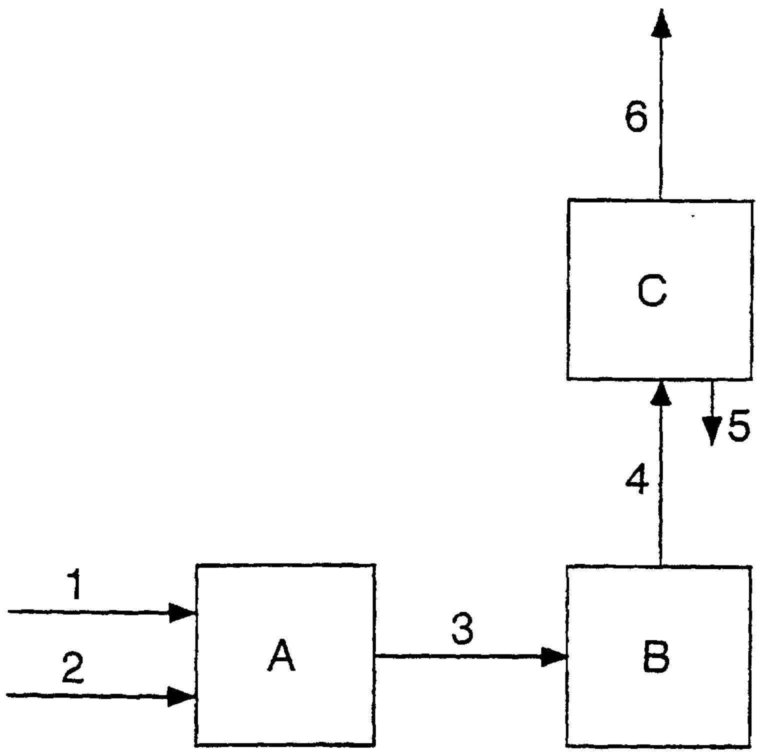

FIG.2